# EUROPÄISCHE PATENTSCHRIFT

(11) **EP 1 943 966 B1**
(45) Veröffentlichungstag und Bekanntmachung des Hinweises auf die Patenterteilung: **14.04.2010**
(21) Anmeldenummer: 07025245.7
(22) Anmeldetag: 31.12.2007
(51) Int. Cl.: A61B 17/16, B23B 51/04

(54) **Werkzeug für das Einbringen von Bohrungen in Knochen oder das Entnehmen von zylindrischen Bohrkernen aus Knochen des menschlichen Körpers**
Tool for creating drill holes in bones or removing cylindrical drill cores from bones in the human body
Outil destiné à l'introduction de perçages dans des os ou l'extraction de carottages cylindriques à partir d'os du corps humain

(30) Priorität: 09.01.2007 DE 102007002089
(43) Veröffentlichungstag der Anmeldung: 16.07.2008
(73) Patentinhaber: REINHARD Feinmechanik GmbH, 63128 Dietzenbach (DE)
(72) Erfinder: Reinhard, Helmut, Dipl.-Ing., 60386 Frankfurt (DE)
(74) Vertreter: Oppermann, Ewald

(56) Entgegenhaltungen:
- EP-A- 1 447 194
- DE-U1- 8 513 328
- DE-U1-202005 016 761
- GB-A- 2 310 623
- JP-A- 4 105 811
- JP-A- 9 117 814

## Beschreibung

### TECHNISCHES GEBIET

Die Erfindung bezieht sich auf ein Werkzeug für das Einbringen von Bohrungen in Knochen oder das Entnehmen von zylindrischen Bohrkernen aus Knochen des menschlichen Körpers entsprechend dem Oberbegriff des Patentanspruchs 1.

### STAND DER TECHNIK

In der rekonstruktiven Chirurgie finden für das Einbringen von Bohrungen in und das Gewinnen von zylindrischen Bohrkernen aus Knochen schneidend- bzw. schleifendwirkende, rotierend angetriebene Werkzeuge Anwendung. Für den Operateur ist eine Bewertung der Schneid- oder Schleifelemente der Werkzeuge im Hinblick auf eine ausreichende Schneid- bzw. Schleifleistung der Werkzeuge nicht immer eindeutig möglich. Um eine ausreichende Schneid- bzw. Schleifleistung zu erzielen sollen daher die Werkzeuge nur einmal zum Einsatz kommen. Die bekannten Werkzeugausführungen können aber beliebig oft mit der Werkzeugaufnahme der Antriebsvorrichtung fest verbunden und davon wieder gelöst werden, so daß eine Einmalverwendung nicht sichergestellt ist. DE 202005016761 U1 zeigt ein Werkzeug gemäß dem Oberbegriff des Anspruchs 1.

### AUFGABENSTELLUNG

Der Erfindung liegt die Aufgabe zugrunde, ein Werkzeug bereitzustellen, welches als Einwegwerkzeug ausgebildet ist und bei geringen Herstellkosten und geringem Bauraumbedarf eine nur einmalige sichere Verbindung mit der Antriebsvorrichtung ermöglicht.

### DARSTELLUNG DER ERFINDUNG

Diese Aufgabe wird erfindungsgemäß durch die Merkmale des Patentanspruchs 1 gelöst. Vorteilhafte oder zweckmäßige Weiterbildungen gehen aus den Unteransprüchen hervor und sind nachfolgend ebenfalls näher beschrieben.

Das erfindungsgemäße Werkzeug ist dadurch gekennzeichnet, daß der Werkzeugkörper an seinem inneren Ende eine sich bis zur Endfläche des Werkzeugkörpers erstreckende Aussparung aufweist, in welche ein aus der Innenwandfläche der Werkzeugaufnahme vorstehender Vorsprung aufgenommen ist, wenn der Werkzeugkörper bis zu einem Anschlag in die Werkzeugaufnahme eingeführt ist, und daß axial von der Aussparung beabstandet eine aus der Wandung des Werkzeugkörpers freigeschnittene und zum äußeren Ende des Werkzeugkörpers weisende und nach außen ausgestellte Zunge vorgesehen ist, die beim Einschieben des Werkzeugkörpers in die Werkzeugaufnahme unmittelbar vor dem Auftreffen des Werkzeugkörpers auf den Anschlag in eine ihr zugeordnete Öffnung in der Wand der Werkzeugaufnahme, den Werkzeugkörper in der Werkzeugaufnahme gegen Axialverschiebungen sperrend, eintritt, aus der sie zur Entfernung des Werkzeugkörpers aus der Werkzeugaufnahme nur unter bleibender Deformation der Zunge herausbiegbar ist.

Bei dem erfindungsgemäßen Werkzeug sind diejenigen Elemente, welche die drehfeste Verbindung zwischen dem Werkzeugkörper und der Werkzeugaufnahme herstellen, nämlich die Aussparung im Werkzeugkörper und der Vorsprung der Werkzeugaufnahme, von denjenigen Elementen, welche einmalig eine axiale Festlegung des Werkzeugkörpers in der Werkzeugaufnahme herbeiführen, nämlich die biegbare Zunge des Werkzeugkörpers und die dieser zugeordnete Öffnung in der Wand der Werkzeugaufnahme, funktionell und räumlich voneinander getrennt an dem Werkzeugkörper bzw. der Werkzeugaufnahme angeordnet. Wenn der Werkzeugkörper in die Werkzeugaufnahme bis zum Anschlag eingeführt ist, ist er drehfest an die Werkzeugaufnahme angeschlossen und wird von dieser bei entsprechender Betätigung der Antriebsvorrichtung drehend angetrieben. Wegen des Eingriffs der Zunge in die zugeordnete Öffnung in der Wand der Werkzeugaufnahme kann der Werkzeugkörper nicht aus der Werkzeugaufnahme axial herausgezogen werden und das Werkzeug ist einsatzbereit. Diese axiale Sperre kann nur durch Druckeinwirkung auf die Zunge von außen her aufgehoben werden, wobei die Zunge plastisch so verformt wird, daß sie in die Öffnung in der Wand der Werkzeugaufnahme nicht zurückfedern kann. Bei dem dadurch unbrauchbar gemachten Werkzeug kann zwar der Werkzeugkörper aus der Werkzeugaufnahme herausgezogen und auch wieder eingeführt werden, jedoch ist er wegen der unbrauchbar gewordenen Zunge nicht mehr axial in der Werkzeugaufnahme fixierbar. Die bei der Fertigung des Werkzeugs nach außen ausgestellte Zunge wird beim Einführen des Werkzeugkörpers in die Werkzeugaufnahme teils elastisch und teils schon plastisch in die Wandung des Werkzeugkörpers zurückgebogen, so daß der Werkzeugkörper in seine Werkzeugaufnahme eingeführt werden kann, wobei die Zunge bei Erreichen der Öffnung in der Werkzeugaufnahme in diese Öffnung elastisch einfedert.

Vorteilhaft ist in Weiterführung des Erfindungsgedankens die Anordnung so getroffen, daß die Zunge im Bereich ihrer Zungenwurzel durch Anbringung einer durch die Zungenwandung hindurchgeführten Ausnehmung eine verringerte Querschnittsfläche besitzt. Je nach Größe und geometrischer Form dieser Öffnung verbleiben zu beiden Seiten der Ausnehmung bis zum Zungenrand nur zwei schmale Stege aus der Wand des ohnehin dünnwandigen rohrförmigen Werkzeugkörpers, so daß die Zunge vor der Entfernung des Werkzeugs aus der Werkzeugaufnahme leicht aus der Öffnung in die Werkzeugaufnahme von außen her herausgebogen und in das Innere des rohrförmigen Werkzeugkörpers bleibend verlagert werden kann. Die zu Beginn des Herausbiegevorgangs etwa noch vorhandene Restelastizität der Zunge wird beim Herausbiegen schnell in eine plastische, d.h. bleibende Zungenverformung überführt, so daß das damit unbrauchbar gewordene Werkzeug aus seiner Werkzeugaufnahme herausgezogen und entsorgt werden kann. Bei geeigneter Werkstoffwahl für den Werkzeugkörper, vorzugsweise wird dieser aus einem korrosionsbeständigen austenitischen oder martensitischen Stahl gefertigt, und bei entsprechender Größe und Form der Ausnehmung in der Zungenwurzel sowie ausreichend großem Herausbiegeweg kann die Zunge beim Herausbiegevorgang im Bereich der beiden dünnen Stege sogar abbrechen, was durchaus im Sinne der dann vorliegenden Unbrauchbarkeit des Werkzeugs erwünscht ist.

Zweckmäßig ist der aus der Innenwandfläche der Werkzeugaufnahme vorstehende Vorsprung Teil eines radial in einer Bohrung der Werkzeugaufnahme befestigten Rundbolzens, während die den Vorsprung aufnehmende Aussparung des Werkzeugkörpers U-förmig ausgebildet ist, mit zwei zueinander und zur Längsachse des Werkzeugkörpers parallelen Wandflächen. Der Rundbolzen kann mit der Werkzeugaufnahme durch Schweißen oder Löten untrennbar verbunden sein. Seine runde Querschnittsform erleichtert das Aufschieben der U-förmigen Aussparung des Werkzeugkörpers bei Einführung des Werkzeugkörpers in die Werkzeugaufnahme.

Die der Zunge zugeordnete Öffnung in der Wand der Werkzeugaufnahme kann als Langloch mit zwei zueinander und zur Längsachse der Werkzeugaufnahme parallelen Wandflächen ausgebildet sein. Hierbei ist die Zunge von außen durch die Öffnung hindurch in voller Länge und Breite sichtbar, so daß die Zunge für den Eingriff eines Werkzeugs beim Herausbiegevorgang freiliegt.

In einer bevorzugten Variante ist die der Zunge zugeordnete Öffnung in der Wand der Werkzeugaufnahme als Innennutabschnitt mit zwei zueinander und zur Längsachse der Werkzeugaufnahme parallelen Wandflächen ausgebildet, wobei der Innennutabschnitt mit der Außenfläche der Werkzeugaufnahme nur über eine radial angeordnete Eingriffsbohrung in Verbindung steht. Diese Variante bietet gegenüber der zuvor beschriebenen Anordnung zwei wesentliche Vorteile. Zum einen ist im Hinblick auf die gewünschte Schaffung eines Einwegwerkzeugs ein etwaiges Manipulieren der Zunge durch den Anwender durch den engen Querschnitt der Eingriffsbohrung hindurch ausgeschlossen. Zum anderen strömt durch den vergleichsweise kleineren Querschnitt der Eingriffsbohrung und den damit verbundenen höheren Strömungswiderstand eine geringere Leckagemenge an Wasser, welches als Kühlflüssigkeit durch den Werkzeugkörper strömt, seitlich ab.

Vorzugsweise sind die Aussparung des Werkzeugkörpers mit dem zugehörigen Vorsprung der Werkzeugaufnahme einerseits und die Zunge des Werkzeugkörpers mit der zugeordneten Öffnung in der Wand der Werkzeugaufnahme andererseits in axialer Richtung miteinander fluchtend hintereinanderliegend angeordnet. Das Werkzeug kann aber auch so ausgebildet sein, daß die Aussparung des Werkzeugkörpers mit dem zugehörigen Vorsprung der Werkzeugaufnahme gegenüber der Zunge des Werkzeugkörpers mit der zugeordneten Öffnung in der Wand der Werkzeugaufnahme um die gemeinsame Längsachse von Werkzeugkörper und Werkzeugaufnahme um einen Winkelbetrag verdreht zueinander angeordnet sind. Hierbei kann jeder die Handhabung des Werkzeugs nicht erschwerender ggf. sogar verbessernder Winkelbetrag gewählt werden, sofern sichergestellt ist, daß er für den Werkzeugkörper und für die Werkzeugaufnahme von gleicher Größe ist.

Die Dimensionierung der miteinander eingreifenden Teile von Werkzeugkörper und Werkzeugaufnahme in Umfangsrichtung ist zweckmäßig so vorzunehmen, daß bei Antriebsanlage des Vorsprungs der Werkzeugaufnahme an die betreffende Wandfläche der Aussparung des Werkzeugkörpers zwischen der Zunge und der ihr in Antriebsdrehrichtung nächstliegenden Wandfläche des Langlochs bzw. des Innennutabschnitts ein Spalt vorgesehen ist. Hierdurch wird sichergestellt, daß das von der Werkzeugaufnahme auf den Werkzeugkörper ausgeübte Drehmoment nur von dem Vorsprung und nicht durch die Zunge auf den Werkzeugkörper übertragen wird.

### KURZE BESCHREIBUNG DER ZEICHNUNGEN

Die Erfindung wird nachfolgend in einem Ausführungsbeispiel anhand der gegenüber den tatsächlichen Abmessungen vergrößerten und zum Teil schematisierten Zeichnungen näher erläutert. Darin zeigt:
- Fig. 1: ein in eine Werkzeugaufnahme betriebsfertig eingeführtes Werkzeug im wesentlichen in Längsschnittdarstellung, mit einer als Langloch ausgebildeten, die Zunge aufnehmenden Öffnung in der Wand der Werkzeugaufnahme,
- Fig. 2: die in Fig. 1 dargestellte Anordnung in teilweise aufgebrochener Draufsicht,
- Fig. 3: die Schnittansicht entsprechend der Schnittverlaufslinie III-III in Fig. 1,
- Fig. 4: die Draufsicht auf das Werkzeug,
- Fig. 5: eine der Fig. 1 ähnliche Längsschnittdarstellung, jedoch mit als Innennutabschnitt ausgebildeter Variante der die Zunge aufnehmenden Öffnung in der Wand der Werkzeugaufnahme,
- Fig. 6: die in Fig. 5 dargestellte Anordnung in teilweise aufgebrochener Draufsicht und
- Fig. 7: die Querschnittsansicht entsprechend der Schnittverlaufslinie VII-VII in Fig. 5.

### DETAILLIERTE BESCHREIBUNG DES AUSFÜHRUNGSBEISPIELS

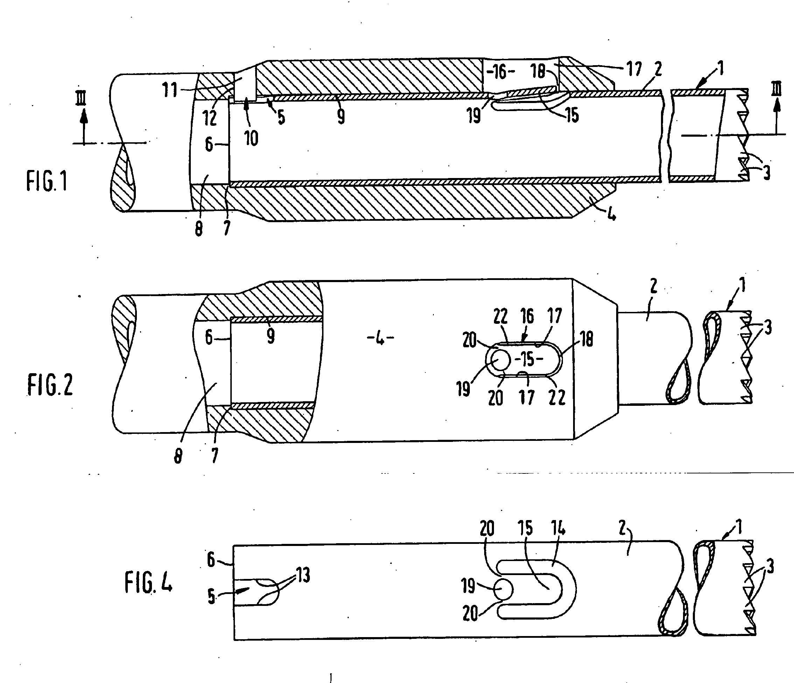

Zunächst wird auf die Fig. 1 bis 4 Bezug genommen. Wie daraus zu erkennen ist, besteht das allgemein mit der Bezugszahl 1 bezeichnete Werkzeug aus einem dünnwandigen rohrförmigen Werkzeugkörper 2 aus Stahl, der an seinem äußeren Ende mit Schneid-oder Schleifelementen 3 versehen ist. Im gezeigten Beispiel handelt es sich um zahnähnliche Schneidelemente. Der Werkzeugkörper 2 ist in eine ebenfalls rohrförmig ausgebildete Werkzeugaufnahme 4 axial eingeführt, welche den Werkzeugkörper 2 eng umschließt, jedoch Verschiebebewegungen des Werkzeugkörpers 2 beim Einführen und Herausziehen in die Werkzeugaufnahme bzw. aus der Werkzeugaufnahme zuläßt. Die an ihrem inneren Ende abgebrochen dargestellte Werkzeugaufnahme 4 ist mit einer Antriebsvorrichtung (nicht dargestellt) für rotierenden Antrieb der Werkzeugaufnahme 4 und damit des Werkzeugs 1 verbindbar.

Am inneren Ende des Werkzeugkörpers 2 ist eine Aussparung 5 vorgesehen, die durch die Wanddicke des Werkzeugkörpers 2 hindurchgeht und sich bis zur Endfläche 6 des Werkzeugkörpers 2 erstreckt, d.h. nach dieser Seite hin offen ist. Bei vollständiger Einführung des Werkzeugkörpers liegt diese Endfläche 6 wegbegrenzend einem als Ringstufe ausgebildeten Anschlag 7 in der zylindrischen Aufnahmebohrung 8 der Werkzeugaufnahme 4 an.

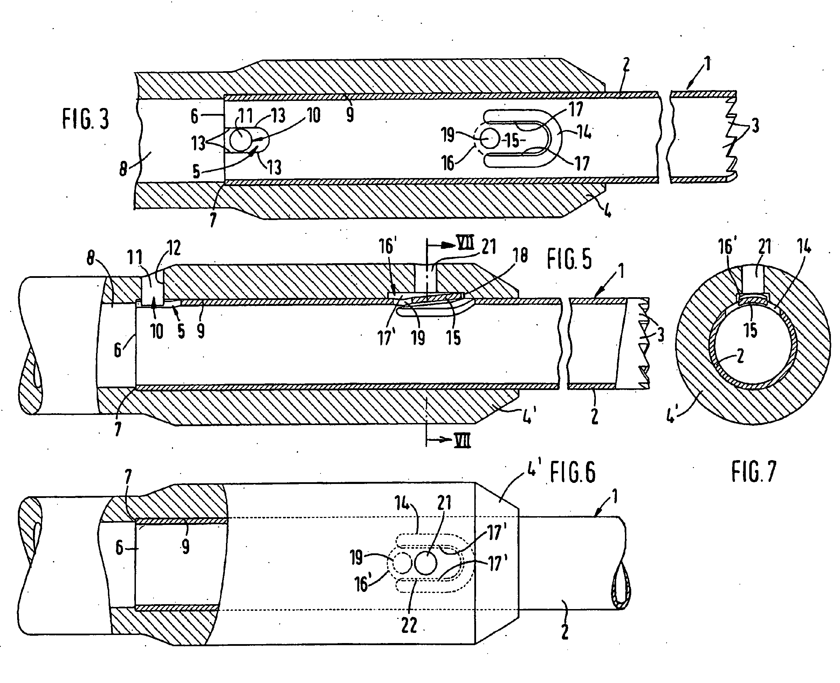

Aus der Innenwandfläche 9 der Werkzeugaufnahme 4 steht ein Vorsprung 10 in die Aufnahmebohrung 8 vor, welcher von der Aussparung 5 aufgenommen ist, wenn der Werkzeugkörper 2 bis zum Anschlag 7 in die Werkzeugaufnahme 4 eingeführt ist. Der aus der Innenwandfläche 9 der Werkzeugaufnahme 4 vorstehende Vorsprung 10 ist Teil eines Rundbolzens 11, der in einer Bohrung 12 der Werkzeugaufnahme 4 befestigt ist. Die den Vorsprung 10 aufnehmende Aussparung 5 des Werkzeugkörpers 2 ist U-förmig ausgebildet, wie aus den Fig. 3 und 4 hervorgeht, und besitzt zwei zueinander und zur Längsachse des Werkzeugkörpers 2 parallele Wandflächen 13.

Wie am besten aus Fig. 4 hervorgeht, ist axial von der Aussparung 5 beabstandet eine aus der Wandung des Werkzeugkörpers 2 mittels eines U-förmigen Freischnitts 14 gebildete Zunge 15 vorgesehen, die zum äußeren Ende des Werkzeugkörpers 2 weisend angeordnet und unter plastischer Materialverformung nach außen ausgestellt ist, wie Fig. 1 verdeutlicht. Der Zunge 15 gegenüberliegend befindet sich eine der Zunge zugeordnete Öffnung 16 in der Wand der Werkzeugaufnahme 4. Diese durch die Wanddicke durchgehende Öffnung 16 ist als Langloch mit zwei zueinander und zur Längsachse der Werkzeugaufnahme 4 parallelen Wandflächen 17 ausgebildet. Die Breite der Öffnung 16, d.h. der Abstand zwischen den Wandflächen 17, ist größer als die Breite der Zunge 15.

Beim Einführen des Werkzeugkörpers 2 in die Aufnahmebohrung 8 der Werkzeugaufnahme 4 tritt die dabei in die Wandung des Werkzeugkörpers 2 zurückgebogene Zunge 15 unmittelbar vor dem Auftreffen der Endfläche 6 des Werkzeugkörpers 2 auf den Anschlag 7 in die ihr zugeordnete Öffnung 16 in der Wand der Werkzeugaufnahme 4 ein, wodurch der Werkzeugkörper 2 in der Werkzeugaufnahme 4 gegen Herausziehen aus der Werkzeugaufnahme 4 gesperrt ist. Wenn die Endfläche 6 dem Anschlag 7 anliegt, wie in Fig. 1 gezeigt ist, befindet sich zwischen dem vorderen freien Zungenende und dem benachbarten gerundeten Wandungsabschnitt der Öffnung 16 ein schmaler Spalt 18 (Fig. 2).

Soll das Werkzeug 1 nach Gebrauch aus der Werkzeugaufnahme 4 entfernt werden, so wird von außen her in die Öffnung 16 ein zum Niederdrücken der Zunge 15 geeigneter Gegenstand eingeführt, mit dessen Hilfe die Zunge 15 aus der Öffnung 16 nach unten in das Innere des Werkzeugkörpers 2 herausgebogen wird, wobei die dabei auftretende plastische Verformung im Bereich der Zungenwurzel hinreichend groß ist, so daß bei Beendigung der Krafteinwirkung auf die Zunge 15 diese nicht wieder sperrend in die Öffnung 16 eintreten kann. Das Einwegwerkzeug 1 kann danach aus der Werkzeugaufnahme 4 herausgezogen werden und ist für weitere Bohrungsverwendungen unbrauchbar gemacht.

Um den Herausbiegevorgang zu erleichtern besitzt die Zunge 15 im Bereich ihrer Zungenwurzel durch Anbringung einer durch die Zungenwandung hindurchgeführten Ausnehmung 19 eine verringerte Querschnittsfläche, so daß zwischen der Ausnehmung 19 und dem Zungenrand nur zwei schmale Stege 20 verbleiben, die leicht verformbar sind.

Die in den Fig. 5 bis 7 gezeigte Variante des bisher unter Bezugnahme auf die Fig. 1 bis 4 beschriebenen Ausführungsbeispiels unterscheidet sich nur im Bereich des Sperreingriffs der Zunge 15 mit der Werkzeugaufnahme. Hierbei ist die der Zunge 15 zugeordnete Öffnung 16' in der Wand der Werkzeugaufnahme 4' nicht durch die volle Wanddicke hindurchgeführt, sondern bildet lediglich einen nach innen offenen Innennutabschnitt mit zwei zueinander und zur Längsachse der Werkzeugaufnahme 4' parallelen Wandflächen 17'. Diese als Innennutabschnitt ausgebildete Öffnung 16' steht mit der Außenfläche der Werkzeugaufnahme 4' nur über eine radial angeordnete Eingriffsbohrung 21 in Verbindung. Diese vergleichsweise enge Eingriffsbohrung 21 verhindert unerwünschte Manipulationen an der einmal aus der Öffnung 16' herausgebogenen Zunge 15 und erlaubt nur das Herausbiegen der Zunge 15 aus der Öffnung 16' mittels eines stiftförmigen in die Eingriffsbohrung 21 passenden Gegenstandes.

Bei dem beschriebenen Ausführungsbeispiel mit seinen beiden Varianten im Bereich des in axialer Richtung sperrenden Zungeneingriffs mit der Werkzeugaufnahme 4 bzw. 4' sind die Aussparung 5 des Werkzeugkörpers 2 mit dem zugehörigen Vorsprung 10 der Werkzeugaufnahme 4, 4' einerseits und die Zunge 15 des Werkzeugkörpers 2 mit der zugeordneten Öffnung 16 bzw. 16' in der Wand der Werkzeugaufnahme 4, 4' andererseits in axialer Richtung miteinander fluchtend hintereinanderliegend angeordnet. Die Anordnung kann aber auch wie eingangs beschrieben so getroffen sein, daß die Aussparung 5 mit dem Vorsprung 10 einerseits gegenüber der Zunge 15 mit der zugeordneten Öffnung 16, 16' andererseits um die gemeinsame Längsachse von Werkzeugkörper 2 und Werkzeugaufnahme 4, 4' um einen Winkelbetrag verdreht zueinander angeordnet sind.

Bei der in Fig. 3 gezeigten Antriebsanlage des Vorsprungs 10 der Werkzeugaufnahme 4 an die betreffende Wandfläche 13 der Aussparung 5 des Werkzeugkörpers 2 ist zwischen der Zunge 15 und der ihr in Antriebsdrehrichtung nächstliegenden Wandfläche 17 der Öffnung 16 ein Spalt 22 vorgesehen. Dieser Spalt 22 verhindert eine Anlage des Randes der Zunge 15 an eine Wandfläche 17, so daß eine Drehmitnahme des Werkzeugs 1 über die Zunge 15 ausgeschlossen ist.

### BEZUGSZEICHENLISTE

- 1: Werkzeug
- 2: Werkzeugkörper
- 3: Schneid- oder Schleifelemente
- 4, 4': Werkzeugaufnahme
- 5: Aussparung
- 6: Endfläche
- 7: Anschlag
- 8: Aufnahmebohrung
- 9: Innenwandfläche
- 10: Vorsprung
- 11: Rundbolzen
- 12: Bohrung
- 13: Wandflächen
- 14: Freischnitt
- 15: Zunge
- 16, 16': Öffnung
- 17, 17': Wandflächen
- 18: Spalt
- 19: Ausnehmung
- 20: Stege
- 21: Eingriffsbohrung
- 22: Spalt

## Patentansprüche

1. Werkzeug (1) für das Einbringen von Bohrungen in Knochen oder das Entnehmen von zylindrischen Bohrkernen aus Knochen des menschlichen Körpers, bestehend aus einem rohrförmigen und um seine Längsachse rotierend antreibbaren Werkzeugkörper (2), der drehfest und axial unverschiebbar mit einer ihn eng umschließenden Werkzeugaufnahme (4) der Antriebsvorrichtung verbindbar ist und an seinem äußeren Ende mit Schneid- oder Schleifelementen (3) versehen ist, **dadurch gekennzeichnet, daß** der Werkzeugkörper (2) an seinem inneren Ende eine sich bis zur Endfläche (6) des Werkzeugkörpers (2) erstreckende Aussparung (5) aufweist, in welche ein aus der Innenwandfläche der Werkzeugaufnahme vorstehender Vorsprung (10) aufgenommen ist, wenn der Werkzeugkörper (2) bis zu einem Anschlag (7) in die Werkzeugaufnahme (4) eingeführt ist, und daß axial von der Aussparung (5) beabstandet eine aus der Wandung des Werkzeugkörpers (2) freigeschnittene und zum äußeren Ende des Werkzeugkörpers (2) weisende und nach außen ausgestellte Zunge (15) vorgesehen ist, die beim Einschieben des Werkzeugkörpers (2) in die Werkzeugaufnahme (4) unmittelbar vor dem Auftreffen des Werkzeugkörpers (2) auf den Anschlag (7) in eine ihr zugeordnete Öffnung (16) in der Wand der Werkzeugaufnahme (4), den Werkzeugkörper (2) in der Werkzeugaufnahme (4) gegen Axialverschiebungen sperrend, eintritt, aus der sie zur Entfernung des Werkzeugkörpers (2) aus der Werkzeugaufnahme (4) nur unter bleibender Deformation der Zunge (15) herausbiegbar ist.

2. Werkzeug nach Anspruch 1, **dadurch gekennzeichnet, daß** die Zunge (15) im Bereich ihrer Zungenwurzel durch Anbringung einer durch die Zungenwandung hindurchgeführten Ausnehmung (19) eine verringerte Querschnittsfläche besitzt.

3. Werkzeug nach Anspruch 1 oder 2, **dadurch gekennzeichnet, daß** der aus der Innenwandfläche (9) der Werkzeugaufnahme (4) vorstehende Vorsprung (10) Teil eines radial in einer Bohrung (12) der Werkzeugaufnahme (4) befestigten Rundbolzens (11) ist, und die den Vorsprung (10) aufnehmende Aussparung (5) des Werkzeugkörpers (2) U-förmig ausgebildet ist, mit zwei zueinander und zur Längsachse des Werkzeugkörper (2) parallelen Wandflächen (13).

4. Werkzeug nach einem der Ansprüche 1 bis 3, **dadurch gekennzeichnet, daß** die der Zunge (15) zugeordnete Öffnung (16) in der Wand der Werkzeugaufnahme (4) als Langloch mit zwei zueinander und zur Längsachse der Werkzeugaufnahme (4) parallelen Wandflächen (17) ausgebildet ist.

5. Werkzeug nach einem der Ansprüche 1 bis 3, **dadurch gekennzeichnet, daß** die der Zunge (15) zugeordnete Öffnung (16') in der Wand der Werkzeugaufnahme (4') als Innennutabschnitt mit zwei zueinander und zur Längsachse der Werkzeugaufnahme (4') parallelen Wandflächen (17') ausgebildet ist, wobei die als Innennutabschnitt ausgebildete Öffnung (16') mit der Außenfläche der Werkzeugaufnahme (4') nur über eine radial angeordnete Eingriffsbohrung (21) in Verbindung steht.

6. Werkzeug nach einem der Ansprüche 1 bis 5, **dadurch gekennzeichnet, daß** die Aussparung (5) des Werkzeugkörpers (2) mit dem zugehörigen Vorsprung (10) der Werkzeugaufnahme (4, 4') einerseits und die Zunge (15) des Werkzeugkörpers (2) mit der zugeordneten Öffnung (16, 16') in der Wand der Werkzeugaufnahme (4, 4') andererseits in axialer Richtung miteinander fluchtend hintereinanderliegend angeordnet sind.

7. Werkzeug nach einem der Ansprüche 1 bis 5, **dadurch gekennzeichnet, daß** die Aussparung (5) des Werkzeugskörpers (2) mit dem zugehörigen Vorsprung (10) der Werkzeugaufnahme (4, 4') gegenüber der Zunge (15) des Werkzeugkörpers (2) mit der zugeordneten Öffnung (16, 16') in der Wand der Werkzeugaufnahme (4, 4') um die gemeinsame Längsachse von Werkzeugkörper (2) und Werkzeugaufnahme (4, 4') um einen Winkelbetrag verdreht zueinander angeordnet sind.

8. Werkzeug nach einem der Ansprüche 1 bis 7, **dadurch gekennzeichnet, daß** bei Antriebsanlage des Vorsprungs (10) der Werkzeugaufnahme (4, 4') an die betreffende Wandfläche (13) der Aussparung (5) des Werkzeugkörpers (2) zwischen der Zunge (15) und der ihr in Antriebsdrehrichtung nächstliegenden Wandfläche (17, 17') der Öffnung (16, 16') ein Spalt (22) vorgesehen ist.

## Claims

1. Tool (1) for making drill-holes in bones or removing cylindrical drill-hole cores from bones of the human body, consisting of a tubular tool body (2) which can be driven in rotation about its longitudinal axis, which tool body can be connected in a rotationally fixed and axially non-displaceable manner to a tool holder (4) of the drive device which tightly surrounds it, and is provided with cutting or grinding elements (3) at its outer end, **characterized in that** the tool body (2) has at its inner end a cutout (5) which extends as far as the end face (6) of the tool body (2), in which cutout a protrusion (10) which protrudes from the inner wall surface of the tool holder is received when the tool body (2) is inserted into the tool holder (4) as far as a stop (7), and **in that** an outwardly projecting tongue (15) which is cut out of the wall of the tool body (2) and points towards the outer end of the tool body (2) is provided at an axial distance from the cutout (5), which tongue, when the tool body (2) is pushed into the tool holder (4) and immediately before the tool body (2) reaches the stop (7), enters an assigned opening (16) in the wall of the tool holder (4), in a manner such as to prevent any axial displacements of the tool body (2) in the tool holder (4), from which opening it can be bent out only with permanent deformation of the tongue (15) in order to remove the tool body (2) from the tool holder (4).

2. Tool according to claim 1, **characterized in that** the tongue (15) has a reduced cross-sectional area in the region of its tongue root due to the provision of a hole (19) through the tongue wall.

3. Tool according to claim 1 or 2, **characterized in that** the protrusion (10) protruding from the inner wall surface (9) of the tool holder (4) is part of a round bolt (11) fixed radially in a bore (12) of the tool holder (4), and the cutout (5) in the tool body (2) which receives the protrusion (10) is U-shaped with two wall surfaces (13) parallel to one another and to the longitudinal axis of the tool body (2).

4. Tool according to one of claims 1 to 3, **characterized in that** the opening (16) in the wall of the tool holder (4) which is assigned to the tongue (15) is designed as an elongate hole with two wall surfaces (17) parallel to one another and to the longitudinal axis of the tool holder (4).

5. Tool according to one of claims 1 to 3, **characterized in that** the opening (16') in the wall of the tool holder (4') which is assigned to the tongue (15) is designed as an inner groove section with two wall surfaces (17') parallel to one another and to the longitudinal axis of the tool holder (4'), wherein the opening (16') designed as an inner groove section is connected to the outer surface of the tool holder (4') only via a radially arranged access bore (21).

6. Tool according to one of claims 1 to 5, **characterized in that** the cutout (5) in the tool body (2) with the associated protrusion (10) of the tool holder (4, 4') on the one hand and the tongue (15) of the tool body (2) with the associated opening (16, 16') in the wall of the tool holder (4, 4') on the other hand are arranged one behind the other and aligned with one another in the axial direction.

7. Tool according to one of claims 1 to 5, **characterized in that** the cutout (5) in the tool body (2) with the associated protrusion (10) of the tool holder (4, 4'), relative to the tongue (15) of the tool body (2) with the associated opening (16, 16') in the wall of the tool holder (4, 4'), are arranged rotated through an angle with respect to one another about the common longitudinal axis of the tool body (2) and tool holder (4, 4').

8. Tool according to one of claims 1 to 7, **characterized in that**, when the protrusion (10) of the tool holder (4, 4') bears against the relevant wall surface (13) of the cutout (5) in the tool body (2), there is a gap (22) between the tongue (15) and the wall surface (17, 17') of the opening (16, 16') located closest to it in the driven rotation direction.

## Revendications

1. Outil (1) destiné à pratiquer des perçages dans des os ou à réaliser des prélèvements de carottes ou biopsies osseuses cylindriques d'os du corps humain, constitué d'un corps d'outil (2) de forme tubulaire pouvant être entraîné en rotation autour de son axe longitudinal, et qui peut être relié de manière fixe en rotation et axialement non coulissante à un porte-outil (4) du dispositif d'entraînement l'entourant de manière étroite, et est pourvu, à son extrémité extérieure, d'éléments de coupe ou d'abrasion (3), le corps d'outil (2) présentant, à son extrémité intérieure, une encoche (5) qui s'étend jusqu'à la surface terminale (6) du corps d'outil (2) et dans laquelle est reçue une protubérance (10) faisant saillie de la surface de paroi intérieure du porte-outil lorsque le corps d'outil (2) est engagé dans le porte-outil (4) jusqu'à une butée (7), **caractérisé en ce qu'**il est prévu axialement à distance de l'encoche (5), une languette (15) dégagée par découpe dans la paroi du corps d'outil (2) et dirigée vers l'extrémité extérieure du corps d'outil (2) tout en étant coudée vers l'extérieur, cette languette pénétrant, lors de l'insertion du corps d'outil (2) dans le porte-outil (4), dans une ouverture (16) qui lui est associée dans la paroi du porte-outil (4), juste avant l'entrée en contact du corps d'outil (2) avec la butée (7), en assurant ainsi le blocage du corps d'outil (2) dans le porte-outil (4) à l'encontre d'un coulissement axial, et, pour retirer le corps d'outil (2) du porte-outil (4), la languette doit être extraite de l'ouverture associée, par pliage, entraînant obligatoirement la déformation permanente de la languette (15).

2. Outil selon la revendication 1, **caractérisé en ce que** la languette (15) possède une surface de section transversale diminuée dans la zone de sa base ou racine de languette, grâce à la réalisation d'un évidement (19) traversant la paroi de la languette.

3. Outil selon la revendication 1 ou la revendication 2, **caractérisé en ce que** la protubérance (10) faisant saillie de la surface de paroi intérieure (9) du porte-outil (4) fait partie d'une broche cylindrique (11) fixée radialement dans un alésage (12) du porte-outil (4), et l'encoche (5) du corps d'outil (2), qui reçoit la protubérance (10), est d'une configuration en forme de U et présente deux surfaces de paroi (13) parallèles l'une à l'autre et à l'axe longitudinal du corps d'outil (2).

4. Outil selon l'une des revendications 1 à 3,
**caractérisé en ce que** l'ouverture (16) associée à la languette (15), dans la paroi du porte-outil (4), est réalisée en tant que trou oblong présentant deux surfaces de paroi (17) parallèles l'une à l'autre et à l'axe longitudinal du porte-outil (4).

5. Outil selon l'une des revendications 1 à 3,
**caractérisé en ce que** l'ouverture (16') associée à la languette (15), dans la paroi du porte-outil (4'), est réalisée en tant que tronçon de rainure intérieure présentant deux surfaces de paroi (17') parallèles l'une à l'autre et à l'axe longitudinal du porte-outil (4'), l'ouverture (16') réalisée en tant que tronçon de rainure intérieure n'étant en liaison avec la surface extérieure du porte-outil (4') que par l'intermédiaire d'un alésage d'accès (21) d'orientation radiale.

6. Outil selon l'une des revendications 1 à 5,
**caractérisé en ce que** l'encoche (5) du corps d'outil (2) avec la protubérance (10) associée du porte-outil (4, 4') d'une part, et la languette (15) du corps d'outil (2) avec l'ouverture (16, 16') associée dans la paroi du porte-outil (4, 4') d'autre part, sont agencées les unes à la suite des autres et de manière alignée dans la direction axiale.

7. Outil selon l'une des revendications 1 à 5,
**caractérisé en ce que** l'encoche (5) du corps d'outil (2) avec la protubérance (10) associée du porte-outil (4, 4') sont agencées, par rapport à la languette (15) du corps d'outil (2) avec l'ouverture (16, 16') associée dans la paroi du porte-outil (4, 4'), de manière décalée d'une valeur angulaire autour de l'axe longitudinal commun du corps d'outil (2) et du porte-outil (4, 4').

8. Outil selon l'une des revendications 1 à 7,
**caractérisé en ce que** dans l'état d'appui d'entraînement de la protubérance (10) du porte-outil (4, 4') contre la surface de paroi (13) considérée de l'encoche (5) du corps d'outil (2), il existe un interstice (22) entre la languette (15) et la surface de paroi (17, 17') de l'ouverture (16, 16'), qui est la plus proche de la languette dans le sens de rotation d'entraînement.
